# EUROPÄISCHE PATENTANMELDUNG

(11) **EP 4 245 350 A1**
(43) Veröffentlichungstag der Anmeldung: **20.09.2023**
(21) Anmeldenummer: 22162406.7
(22) Anmeldetag: 16.03.2022
(51) Int. Cl.: A61M 37/00

(54) **MICRONEEDLING-HANDGERÄT, HAUTAPPLIKATIONSEINRICHTUNG UND ARTIKEL**

(71) Anmelder: MT.DERM GmbH, 12307 Berlin (DE)
(72) Erfinder: SCHERKOWSKI, Dirk, 12489 Berlin (DE)
(74) Vertreter: Bittner, Thomas L.

(57) **Zusammenfassung**

Die Erfindung betrifft ein Microneedling-Handgerät (30) mit: einem Gehäuse (2); einer Hautapplikationseinrichtung (6); einem Microneedling-Applikator (8), der an der Hautapplikationseinrichtung (6) gebildet und entlang einer Bewegungsrichtung vor und zurück bewegbar ist; und einer Nadelplatte (9), die an dem Microneedling-Applikator (8) gebildet ist und bei der über eine vorderseitige Anwendungsfläche (11) verteilt mehrere Applikationsnadeln (10) angeordnet sind. Der Microneedling-Applikator (8) ist im Betrieb zwischen einer vorderen Arbeitsstellung und einer im Vergleich hierzu zurückgestellten hinteren Arbeitsstellung vor und zurück bewegbar. Die vorderseitige Anwendungsfläche (11) mit den hierauf verteilt angeordneten mehreren Applikationsnadeln (10) ist, zumindest in der vorderen Arbeitsstellung, zu wenigstens einer Bezugsrichtung aus der folgenden Gruppe schräg gestellt: Längsrichtung des Gehäuses (2), Bewegungsrichtung des Microneedling-Applikators (8) beim Verlagern zwischen der vorderen und der zurückgestellten hinteren Arbeitsstellung und Bewegungsrichtung der Nadelplatte (9) beim Verlagern zwischen der vorderen und der zurückgestellten hinteren Arbeitsstellung. Weiterhin sind eine Hautapplikationseinrichtung (6) sowie ein Artikel geschaffen.

## Beschreibung

Die Erfindung betrifft ein Microneedling-Handgerät, eine Hautapplikationseinrichtung für ein Microneedling-Handgerät und einen Artikel.

### Hintergrund

Microneedling sieht üblicherweise vor, die Haut mit mehreren Stechnadeln in einem flächigen Hautabschnitt gleichzeitig lokal aufzustechen. Die mehreren Stechnadeln sind bei bekannten Microneedling-Hautstechwerkzeugen eines Handgeräts über eine Anwendungsfläche verteilt angeordnet, zum Beispiel an einer Nadelplatte.

Das Microneedling verursacht aufgrund des lokalen Aufstechens feinste Verletzungen der Haut, die die Haut als Wunden registriert. In der Haut werden dann verschiedene Botenstoffe und Wachstumsfaktoren ausgeschüttet, um die Wundheilung anzuregen. Dieser Vorgang regt auch die Produktion von Kollagen, Elastin und Hyaluronsäure an, die Straffung und Elastizität der Haut fördern. Microneedling kann mit und ohne Substanz- oder Wirkstoffeintrag in die Haut ausgeführt werden. Die Haut wird lokal aufgestochen, um positive Heilungsreaktionen auszulösen, was wahlweise mittels Substanzeintrag unterstützt werden kann.

Zum Anwenden des Microneedling sind beispielsweise manuelle Hautstecheinrichtungen in stempelartiger Ausführung bekannt, bei der das Microneedling-Hautstechwerkzeug mit einem Stempel gebildet ist, bei dem über eine Anwendungsfläche verteilt mehrere Stechnadeln angeordnet sind. Bei anderen manuellen Handgeräten sind die Stechnadeln über die Mantelfläche einer Walze verteilt, die zum lokalen Aufstechen der Haut über diese gerollt wird.

Weiterhin sind Microneedling-Handgeräte in Form eines sogenannten Pens bekannt, bei dem ein Microneedling-Hautstechwerkzeug einer Hautstecheinrichtung mit einer Nadelplatte gebildet ist, an der die mehreren Stechnadeln im Bereich einer Anwendungsfläche angeordnet sind. Das Microneedling-Hautstechwerkzeug wird mit Hilfe eines elektrischen Motors einer Antriebseinrichtung automatisiert vor- und zurückbewegt. Mit Hilfe des elektrischen Motors der Antriebseinrichtung wird eine Antriebskraft bereitgestellt, die auf das Microneedling-Hautstechwerkzeug eingekoppelt wird, um die Nadelplatte mit der Anwendungsfläche und den hierauf flächig verteilten Stechnadeln vor und zurück zu bewegen.

Ein Microneedling-Handgerät ist zum Beispiel aus dem Dokument US 2017 / 0354810 A1 bekannt.

### Zusammenfassung

Aufgabe der Erfindung ist es, ein Microneedling-Handgerät zum lokalen Stimulieren und / oder Regenerieren einer Haut anzugeben, welches eine verbesserte Anwendung des Microneedlings ermöglicht.

Gelöst wird die Aufgabe durch ein Microneedling-Handgerät nach Anspruch 1. Weiterhin sind eine Hautapplikationseinrichtung für ein Microneedling-Handgerät sowie ein Artikel nach den nebengeordneten Ansprüchen 15 und 16 geschaffen. Ausgestaltungen sind Gegenstand von abhängigen Unteransprüchen.

Nach einem Aspekt ist ein Microneedling-Handgerät geschaffen, welches Folgendes aufweist: ein Gehäuse; eine Hautapplikationseinrichtung; ein Microneedling-Applikator, der an der Hautapplikationseinrichtung gebildet und entlang einer Bewegungsrichtung vor und zurück bewegbar ist; und eine Nadelplatte, die an dem Microneedling-Applikator gebildet ist und bei der über eine vorderseitige Anwendungsfläche verteilt mehrere Applikationsnadeln angeordnet sind. Der Microneedling-Applikator ist im Betrieb zwischen einer vorderen Arbeitsstellung und einer im Vergleich hierzu zurückgestellten hinteren Arbeitsstellung vor und zurück bewegbar. Die vorderseitige Anwendungsfläche mit den hierauf verteilt angeordneten mehreren Applikationsnadeln ist, zumindest in der vorderen Arbeitsstellung, zu wenigstens einer Bezugsrichtung aus der folgenden Gruppe schräg gestellt: Längsrichtung des Gehäuses, Bewegungsrichtung des Microneedling-Applikators beim Verlagern zwischen der vorderen und der zurückgestellten hinteren Arbeitsstellung und Bewegungsrichtung der Nadelplatte beim Verlagern zwischen der vorderen und der zurückgestellten hinteren Arbeitsstellung.

Nach einem weiteren Aspekt ist eine Hautapplikationseinrichtung mit einem Gehäuse geschaffen, die zum Ausbilden eines Microneedling-Handgeräts an eine Antriebseinrichtung koppelbar ist, wobei die Hautapplikationseinrichtung Folgendes aufweist: ein Microneedling-Applikator, das an der Hautapplikationseinrichtung gebildet ist, derart, dass der Microneedling-Applikator im Betrieb entlang einer Bewegungsrichtung vor und zurück bewegbar ist; und eine Nadelplatte, die an dem Microneedling-Applikator gebildet ist und bei der über eine vorderseitige Anwendungsfläche verteilt mehrere Applikationsnadeln angeordnet sind. Der Microneedling-Applikator ist zwischen einer vorderen Arbeitsstellung und einer im Vergleich hierzu zurückgestellten hinteren Arbeitsstellung vor und zurück bewegbar oder verlagerbar. Die vorderseitige Anwendungsfläche mit den hierauf verteilt angeordneten mehreren Applikationsnadeln ist, zumindest in der vorderen Arbeitsstellung, zu wenigstens einer Bezugsrichtung aus der folgenden Gruppe schräg gestellt: Längsrichtung des Gehäuses, Bewegungsrichtung des Microneedling-Applikators beim Verlagern zwischen der vorderen und der zurückgestellten hinteren Arbeitsstellung und Bewegungsrichtung der Nadelplatte beim Verlagern zwischen der vorderen und der zurückgestellten hinteren Arbeitsstellung.

Nach einem weiteren Aspekt ist ein Artikel geschaffen, der Folgendes aufweist: eine Antriebseinrichtung, die in einem Gehäuse angeordnet ist, welches wahlweise ein Handstück ausbildend ausgeführt ist; einen Zahnbürstenaufsatz, welcher zum Ausbilden einer elektrischen Zahnbürste mit dem Gehäuse lösbar und so an die Antriebseinrichtung koppelnd verbunden werden kann; und eine Hautapplikationseinrichtung, welche zum Ausbilden eines Microneedling-Handgeräts mit dem Gehäuse lösbar und so an die Antriebseinrichtung koppelnd verbunden werden kann.

Der Microneedling-Applikator bildet ein Microneedling-Werkzeug mit der Nadelplatte.

Die Applikationsnadeln können zumindest zum Teil als Stechnadeln mit einer stechenden Nadelspitze ausgeführt sein. Sind alle Applikationsnadeln jeweils als Stechnadel mit stechenden Nadelspitze ausgeführt, ist der Microneedling-Applikator als ein Microneedling-Hautstechwerkzeug gebildet. Mithilfe der stechenden Ausführung der Nadeln (Stechnadeln) ist die Haut, auf welche das Microneedling appliziert wird, lokal aufstechend behandelbar. Es ist dann ein Microneedling-Handgerät zum lokalen Aufstechen einer Haut bereitgestellt.

Die Applikationsnadeln können zumindest zum Teil als Stimulationsnadeln mit einer nicht-stechenden (stumpfer) Nadelspitze ausgeführt sein. Sind alle Applikationsnadeln jeweils als Stimulationsnadel mit nicht-stechender Nadelspitze ausgeführt, ist der Microneedling-Applikator als ein Microneedling-Stimulationswerkzeug gebildet. Mithilfe der nicht-stechenden Ausführung der Nadeln (Stimulationsnadel) ist die Haut, auf welche das Microneedling appliziert wird, lokal nicht-stechend behandelbar. Es ist dann ein Microneedling-Handgerät zum lokalen (nicht-stechenden) Stimulieren und / oder Regenerieren einer Haut bereitgestellt. Mit der nicht-stechenden Ausführung der Nadeln ist ein Handgerät bereitgestellt, welches eine Microneedling-Behandlung (kurz: Microneedling) der Haut ermöglicht, ohne diese lokal aufzustechen, insbesondere für ein Microneedling oder ein Microneedling-ähnliches Behandeln in Hautbereichen, in denen die obere Hautschicht (Epidermis) dünner oder feiner ist als in anderen Hautbereichen, zum Bespiel im Bereich von Hautabschnitte im Gesicht, beispielsweise um die Augen herum.

Auch eine Kombination von Stechnadeln mit stechender Nadelspitze und Stimulationsnadeln mit nicht-stechender (stumpfer) Nadelspitze kann in einem Ausführungsbeispiel vorgesehen sein.

Die Applikationsnadeln können auf der Nadelplatte aufrechtstehend ausgerichtet sein.

Die vorderseitige Anwendungsfläche kann mit der Bezugsrichtung zum Beispiel einen Winkel zwischen etwa 30 Grad und < 90 Grad einschließen, bevorzugt zwischen etwa 30 Grad und etwa 85 Grad und weiter bevorzugt zwischen etwa 45 Grad und etwa 80 Grad.

Das Microneedling-Handgerät kann eine Antriebseinrichtung aufweisen, insbesondere eine motorische Antriebseinrichtung, die in dem Gehäuse angeordnet und eingerichtet ist, eine Antriebskraft bereitzustellen, wobei der Microneedling-Applikator mit der Antriebseinrichtung verbunden ist, derart, dass der Microneedling-Applikator mittels der Antriebskraft im Betrieb mit einer Wiederholfrequenz zwischen der vorderen Arbeitsstellung und der zurückgestellten hinteren Arbeitsstellung vor und zurück bewegbar ist.

Auch eine manuelle (nicht-motorische) Antriebseinrichtung kann vorgesehen sein.

Beim Bewegen zwischen der vorderen Arbeitsstellung und der hinteren Arbeitsstellung können in einer Ausführung zumindest die mehreren Applikationsnadeln der Nadelplatte in der vorderen Arbeitsstellung und der hinteren Arbeitsstellung des Microneedling-Applikators vollständig außerhalb des Gehäuses und so freiliegend angeordnet sein. Das Microneedling-Handgerät ist in einer beispielhaften Ausgestaltung so konstruktiv eingerichtet, ein weiter verbessertes Microneedling zu ermöglichen. Da zumindest die mehreren Applikationsnadeln der Nadelplatte an dem Microneedling-Applikator sowohl in der vorderen Arbeitsstellung wie auch in der hinteren Arbeitsstellung jeweils vollständig außerhalb des wahlweise ein- oder mehrstückigen Gehäuses, insbesondere außerhalb eines Gehäusebauteils der Hautapplikationseinrichtung, und so freiliegend angeordnet sind, kann der lokal zu stimulierende Hautabschnitt aufgrund der repetierenden Bewegung der Nadelplatte mit den mehreren stechenden und / oder nicht-stechenden Applikationsnadeln selbst in Schwingungen versetzt werden, wird also nicht vom Gehäuse des Microneedling-Handgeräts hieran gehindert, was ein effizientes und möglichst schmerzfreies Microneedling mittels der mehreren Applikationsnadeln auf der Hautoberfläche ermöglicht.

Im Betrieb kann in einer beispielhaften Ausführung weiterhin zumindest die vorderseitige Anwendungsfläche sowohl in der vorderen Arbeitsstellung wie auch in der zurückgestellten hinteren Arbeitsstellung des Microneedling-Applikators vollständig außerhalb des Gehäuses und so freiliegend angeordnet sein, insbesondere außerhalb eines von Gehäuseabschnitten des Gehäuses zumindest teilweise umgebenen Gehäuseinnenraum. Auf diese Weise ist das Microneedling-Handgerät ergänzend konstruktiv eingerichtet, im Betrieb beim Microneedling das freie Schwingen des bearbeiteten Hautabschnitts zuzulassen.

Die vorderseitige Anwendungsfläche mit den hierauf verteilt angeordneten mehreren Applikationsnadeln kann sowohl in der vorderen wie auch in der im Vergleich hierzu zurückgestellten hinteren Arbeitsstellung schräg gestellt sein. Hierbei kann die Schräg- oder Neigungsstellung auch während der Bewegung zwischen der vorderen und der hinteren Arbeitsstellung ausgebildet sein.

Bei dem Microneedling-Applikator kann in alternativen Ausgestaltungen die Nadelplatte frei von einem die Nadelplatte zumindest teilweise umgreifenden Gehäuseabschnitt des Gehäuses gebildet sein.

Bei dem Microneedling-Handgerät kann eine Schutz- oder Abdeckkappe vorgesehen sein, die am Gehäuse lösbar und die Nadelplatte abdeckend angeordnet ist, wobei an der Schutzkappe außen ein Funktionswerkzeug angeordnet ist. Zum Beispiel können an der Schutzkappe außen, insbesondere im Bereich einer Fronfläche, mehrere Hautmassagevorsprünge angeordnet sein, die auf der Außenfläche der Schutzkappe verteilt angeordnet sind. Zum Beispiel können die Hautmassagevorsprünge ein abgerundetes Kopfteil aufweisen, beispielsweise mit einem Kugelkopf.

An der Abdeckkappe, welche im aufgesetzten Zustand die mehreren Applikationsnadeln abdeckt, ist so ein Funktionswerkzeug angeordnet, welches mit einer Anordnung von Funktionselementen gebildet sein kann, bei denen es sich beispielsweise um Massageelemente handelt. Die Abdeckkappe ist so als Multifunktionskappe ausführbar.

An der Abdeckkappe können Öffnungen vorgesehen sein. Zum Beispiel sind die Öffnungen im Bereich einer Mantelfläche der Abdeckkappe angeordnet und bilden so seitliche Öffnungen. Alternativ oder ergänzend können solche Öffnungen bei anderen Ausführungsformen im Bereich der Frontfläche angeordnet sein. Zum Beispiel können die Öffnungen dazu dienen, für die Nadelplatte mit den mehreren Applikationsnadeln bei aufgesetzter Abdeckkappe eine Gassterilisation durchzuführen. Seitliche Öffnungen haben den Vorteil, dass der Eintritt von Staubpartikeln zu der Nadelplatte und hierdurch verursachte Staubablagerungen gemindert werden.

Die Nadelplatte kann an einem Gehäuseabschnitt des Gehäuses angeordnet sein, welcher im Betrieb beim Vor- und Zurückbewegen des Microneedling-Applikators mit diesem zusammen mitbewegt wird. Hierbei kann es sich um ein vorderes Gehäuseteil des Gehäuses handeln. Die Nadelplatte kann hierbei auf einer Außenseite des Gehäuseabschnitts gebildet sein, mit der die Anwendungsfläche bereitgestellt ist. Die Nadelplatte kann an einem vorderen Gehäuseabschnitt des Gehäuses angeformt sein, welcher im Betrieb zusammen mit dem Microneedling-Applikator vor und zurück bewegt wird. Beispielsweise kann die Nadelplatte als eine Frontplatte des Gehäuseabschnitts angeformt sein. Die Nadelplatte kann lösbar oder nicht-lösbar an dem Gehäuseabschnitt angeordnet sein.

Die Nadelplatte kann an dem Gehäuseabschnitt des Gehäuses einstückig angeformt sein. Hierbei kann ein seitlicher Wandabschnitt des Gehäuseabschnitts einstückig mit einer die Anwendungsfläche bereitstellenden Frontplatte einstückig ausgeführt sein. Der Gehäuseabschnitt kann so der Microneedling-Applikator bildend ausgeführt sein, insbesondere die Nadelplatte.

Der Microneedling-Applikator kann zusammen mit dem Gehäuseabschnitt von dem Gehäuse lösbar oder abnehmbar sein. Der Microneedling-Applikator und / oder die Hautapplikationseinrichtung mit dem Microneedling-Applikator, sei es separat oder gemeinsam mit dem Gehäuseabschnitt, kann mittels einer Steckverbindung oder einer Schraubverbindung lösbar montiert sein. So kann der Microneedling-Applikator gemeinsam mit dem Gehäuseabschnitt auf einen zugeordneten Gehäuseabschnitt aufsteckbar sein, insbesondere einen Gehäuseabschnitt, der die Antriebseinrichtung aufnimmt.

Bei verschiedenen Ausführungsformen können die mehreren Applikationsnadeln im Bereich der Anwendungsfläche auf die Nadelplatte aufgebracht sein. Alternativ kann vorgesehen sein, dass die Applikationsnadeln in Öffnungen der Nadelplatte angeordnet sind, beispielsweise eingelötet oder eingeklebt sind, wobei sich die Applikationsnadeln zumindest abschnittsweise durch die Öffnungen hindurch erstrecken können. Es kann vorgesehen sein, dass die Applikationsnadeln nach dem Durchgang durch die Öffnungen an einem in Bezug auf die Anwendungsfläche der Nadelplatte rückseitigen Nadelhalterelement aufgenommen sind, welches als Teil des Microneedling-Applikators oder der Nadelplatte ausbildbar ist.

Alternativ können die Applikationsnadeln einstückig an der Nadelplatte angeformt sein. So können die Applikationsnadeln zum Beispiel mittels gestanzter und gebogener Blechelemente gebildet sein.

Applikationsnadeln im Sinne der vorliegenden Offenbarung sind allgemein nicht-scharfe bzw. nicht-stechende oder scharfe bzw. stechende, von der Nadelplatte hervorstehende Volumenelemente, die hinsichtlich Form und Festigkeit für eine wiederholte (stechende / nicht-stechende) Applikation auf die Haut zum Anregen oder Stimulieren der Haut(schichten) eingerichtet sind. Die Applikationsnadeln sind bevorzugt aus einem Vollmaterial, im Unterschied zur Kanüle (Hohlnadel).

Die Applikationsnadeln können Nadeln aus Metall oder Kunststoff sein.

Ein vorderes Gehäuseteil, welches der Hautapplikationseinrichtung zugeordnet ist, kann rückwärtig hülsenförmig gestaltet sein und einen hinteren Gehäuseteil zumindest teilweise umschließen oder umgreifen, welcher der Antriebseinrichtung zugeordnet ist. Bei dieser oder anderen Ausführungsformen kann mit dem vorderen Gehäuseteil, welches vom hinteren Gehäuseteil abnehmbar sein kann, ein Gehäusebauteil der Hautapplikationseinrichtung gebildet sein, zum Beispiel ein Modulgehäuse eines Stimulationsmoduls.

Die Schutzkappe kann auf das vorderseitige Gehäuseende lösbar aufgesetzt sein.

Die Hautapplikationseinrichtung kann an dem Gehäuse lösbar angeordnet sein. Auf diese Weise kann der Microneedling-Applikator mit einem zugeordneten Gehäuseteil wechselbar an dem Gehäuse angeordnet werden.

Die Antriebseinrichtung kann mit einer Antriebseinrichtung für eine elektrische Zahnbürste gebildet sein, welche eingerichtet ist, hieran wahlweise die Hautapplikationseinrichtung oder einen mittels der Antriebseinrichtung betreibbaren Zahnbürstenaufsatz lösbar zu koppeln. Antriebseinrichtungen für elektrische Zahnbürsten sind als solche in verschiedenen Ausführungen bekannt. Das Gehäuse der Antriebseinrichtung bildet häufig ein Handstück der elektrischen Zahnbürste, welches der Nutzer beim Benutzen umgreift. Die Antriebseinrichtung stellt eine Antriebskraft / -bewegung für den elektrischen Betrieb des Zahnbürstenaufsatzes bereit. Der Zahnbürstenaufsatz ist abnehmbar. Die Antriebseinrichtung der elektrischen Zahnbürste kann dann zum Ausbilden des Microneedling-Handgeräts verwendet werden, indem die Hautapplikationseinrichtung auf die Antriebseinrichtung der elektrischen Zahnbürste lösbar aufgesetzt wird, so dass die bereitgestellte Antriebskraft auf der Microneedling-Applikator eingeleitet werden kann, derart, dass der Microneedling-Applikator mittels der Antriebskraft im Betrieb mit einer Wiederholfrequenz vor und zurück bewegt wird.

Der Microneedling-Applikator kann durch eine Öffnung an einem vorderseitigen Gehäuseende des Gehäuses hindurch in eine im Vergleich zur zurückgestellten hinteren Arbeitsstellung weiter zurückgestellte Nichtarbeitsstellung verlagerbar sein, in welcher zumindest die vorderseitige Anwendungsfläche der Nadelplatte gegenüber der Öffnung zurücksteht. Bei dieser Ausführungsform kann der Microneedling-Applikator im Nicht-Betrieb in die zurückgestellte Nichtarbeitsstellung verlagert werden, beispielsweise derart, dass die mehreren Applikationsnadeln hinter einer Öffnungsfläche der (Gehäuse-)Öffnung am vorderseitigen Gehäuseende angeordnet und somit mittels des Gehäuses geschützt sind.

Das Gehäuse kann die Öffnung teilweise oder vollständig umgreifen. In der zurückgestellten Nichtarbeitsstellung kann die Nadelplatte umlaufend teilweise oder vollständig von dem Gehäuse umgeben sein, so dass die Nadelplatte dann im Gehäuseinnenraum angeordnet ist.

Die Nadelplatte kann beim Verlagern durch die (Gehäuse-)Öffnung hindurch ein- oder mehrseitig von einem die Öffnung umgebenden Rand beabstandet sein. Bei dieser Ausführungsform wird die Nadelplatte in dem ein- oder mehrseitig ausgebildeten Abstandsbereichen berührungskontaktfrei durch die Öffnung hindurch verlagert. Insbesondere findet so in den Abstandsbereichen keine Führung der Nadelplatte am Gehäuse statt. Die Beabstandung zwischen der Nadelplatte und dem umgebenden Gehäuse kann um die Nadelplatte herum umlaufend durchgehend ausgebildet sein.

Die Nadelplatte kann in der weiter zurückgestellten Nichtarbeitsstellung beabstandet von die Nadelplatte umgebenden Gehäuseabschnitten des Gehäuses freiliegend angeordnet sein. Die Nadelplatte ist hier berührungskontaktfrei in der weiter zurückgestellten Nichtarbeitsstellung angeordnet.

Das Microneedling-Handgerät kann mit einem wiederverwendbaren Antriebsmodul, in welchem die Antriebseinrichtung angeordnet ist, und einem Einwegmodul gebildet sein, welches mit dem Antriebsmodul lösbar verbunden ist und in dem die Hautapplikationseinrichtung angeordnet ist. Das Antriebsmodul und das Einwegmodul können mit einem jeweils zugeordneten Modulgehäuse gebildet sein, wobei die Modulgehäuse lösbar miteinander gekoppelt oder verbunden werden können.

Die (motorische) Antriebseinrichtung kann eingerichtet sein, der Microneedling-Applikator mittels der hierauf eingeleiteten Antriebskraft im Betrieb mit einer Wiederholfrequenz zwischen etwa 10 Hz und etwa 200 Hz vor und zurück zu bewegen, alternativ mit einer Wiederholfrequenz zwischen etwa 25 Hz und etwa 160 Hz.

Es kann vorgesehen sein, dass zwischen der vorderen Arbeitsstellung und der im Vergleich hierzu zurückgestellten hinteren Arbeitsstellung in der Bewegungsrichtung ein Abstand (Arbeitshublänge) von etwa 0,5 mm bis etwa 5 mm gebildet ist. Alternativ kann vorgesehen sein, dass zwischen der vorderen Arbeitsstellung und der im Vergleich hierzu zurückgestellten hinteren Arbeitsstellung in der Bewegungsrichtung ein Abstand (Arbeitshublänge) von lediglich etwa 1 mm bis etwa 4 mm gebildet ist. Mittels unterschiedlicher Hublängen ist das Microneedling-Handgerät für unterschiedliche Anwendungen konfigurierbar, zum Beispiel für medizinisches oder kosmetisches Microneedling.

Die Nadelplatte kann in einer Ausgestaltung mit einer Wendeplatte oder einem Wendeeinsatz ausgeführt sein, derart, dass unterschiedliche Microneedling-Applikatoren hieran jeweils wechsel- oder lösbar angeordnet werden können, um unterschiedliche (Microneedling-) Funktionen bereitzustellen und / oder einen Verschleiß des Applikators zu kompensieren. Es kann sich auch um Wechseleinsätze handeln, die je nach gewünschter Anwendung an der Nadelplatte lösbar angeordnet werden. Wendeeinsätze können auf beiden Seiten Applikator-Werkzeuge tragen. Die Wechseleinsätze können aus einem Sortiment oder einem abgestuften Satz von Applikatoreigenschaften auswählbar sein.

Die Anwendungsfläche, auf welcher die mehreren Applikationsnadeln angeordnet sind, kann eine konvex gekrümmte Oberfläche aufweisen, welche die Anwendungsfläche ganz oder nur teilweise erfasst, insbesondere nur in einem mittleren Flächenbereich.

Die vorangehend in Verbindung mit dem Microneedling-Handgerät erläuterten Ausgestaltungen können im Zusammenhang mit der Hautapplikationseinrichtung und / oder dem Artikel entsprechend vorgesehen sein.

### Beschreibung von Ausführungsbeispielen

Im Folgenden werden weitere Ausführungsbeispiele unter Bezugnahme auf Figuren einer Zeichnung erläutert. Hierbei zeigen:
- Fig. 1: eine schematische perspektivische Darstellung eines Microneedling-Handgeräts;
- Fig. 2: eine schematische perspektivische Darstellung des Microneedling-Handgeräts aus Fig. 1., wobei ein Stimulationsmodul mit einem Microneedling-Applikator gelöst ist;
- Fig. 3: eine schematische perspektivische Darstellung eines vorderen Abschnitts des Microneedling-Handgeräts aus Fig. 1, wobei der Microneedling-Applikator mit Nadelplatte in einer vorderen Arbeitsstellung angeordnet ist;
- Fig. 4: eine schematische perspektivische Darstellung des vorderen Abschnitts des Microneedling-Handgeräts, wobei der Microneedling-Applikator mit Nadelplatte in einer zurückgestellten hinteren Arbeitsstellung angeordnet ist;
- Fig. 5: eine schematische perspektivische Darstellung des vorderen Abschnitts des Microneedling-Handgeräts, wobei vorderseitig auf der Microneedling-Applikator die Nadelplatte abdeckend eine Abdeck- oder Schutzkappe aufgesetzt ist;
- Fig. 6: eine schematische perspektivische Darstellung des vorderen Abschnitts des Microneedling-Handgeräts teilweise im Schnitt mit aufgesetzter Abdeck- oder Schutzkappe;
- Fig. 7: eine schematische perspektivische Darstellung des vorderen Abschnitts des Microneedling-Handgeräts teilweise im Schnitt mit abgenommener Abdeck- oder Schutzkappe;
- Fig. 8: eine schematische perspektivische Darstellung einer elektrischen Zahnbürste;
- Fig. 9: eine schematische perspektivische Darstellung eines weiteren Microneedling-Handgeräts;
- Fig. 10: eine schematische perspektivische Darstellung eines vorderen Gehäuseteils des Microneedling-Handgeräts aus Fig. 9 im Schnitt;
- Fig. 11: eine schematische perspektivische Darstellung des vorderen Gehäuseteils des Microneedling-Handgeräts aus Fig. 9, wobei die Nadelplatte des Microneedling-Applikators in einer eingefahrenen Nichtarbeitsstellung angeordnet ist;
- Fig. 12: eine schematische perspektivische Darstellung des vorderen Gehäuseteils des Microneedling-Handgeräts aus Fig. 9, wobei die Nadelplatte des Microneedling-Applikators in einer vorderen Arbeitsstellung freiliegend angeordnet ist;
- Fig. 13: eine schematische perspektivische Darstellung des vorderen Gehäuseteils des Microneedling-Handgeräts aus Fig. 9, wobei die Nadelplatte in einer zurückgestellten hinteren Arbeitsstellung freiliegend angeordnet ist;
- Fig. 14: eine schematische perspektivische Darstellung einer Nadelplatte mit einer Anordnung von nicht-stechenden Applikationsnadeln mit stumpfer Nadelspitze im Bereich einer vorderseitigen Anwendungsfläche von schräg oben;
- Fig. 15: eine schematische perspektivische Darstellung von Nadelplatten unterschiedlicher Größe mit einer Anordnung von nicht-stechenden Applikationsnadeln mit stumpfer Nadelspitze im Bereich einer vorderseitigen Anwendungsfläche im Schnitt;
- Fig. 16: eine schematische perspektivische Darstellung eines vorderen Abschnitts eines weiteren Microneedling-Handgeräts teilweise im Schnitt;
- Fig. 17: eine schematische perspektivische Darstellung des vorderen Abschnitts des weiteren Microneedling-Handgeräts aus Fig. 15 mit lösbar aufgesetzter Abdeck- oder Schutzkappe;
- Fig. 18: eine schematische perspektivische Darstellung des vorderen Abschnitts des weiteren Microneedling-Handgeräts aus Fig. 16 mit abgenommener Abdeck- oder Schutzkappe und
- Fig. 19: eine schematische perspektivische Darstellung der Abdeck- oder Schutzkappe.

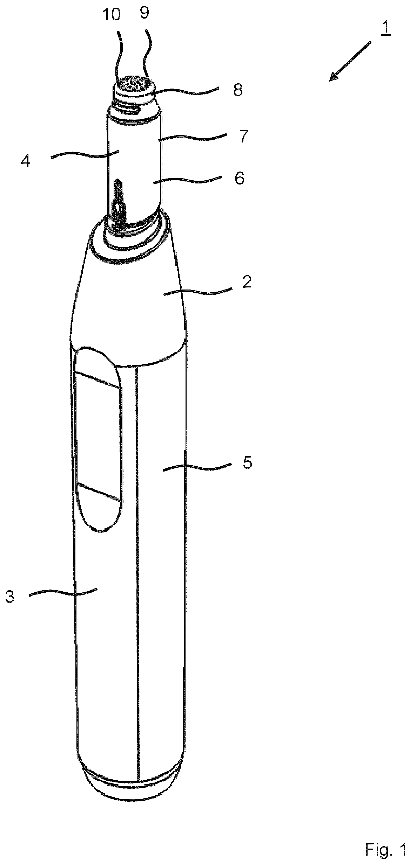

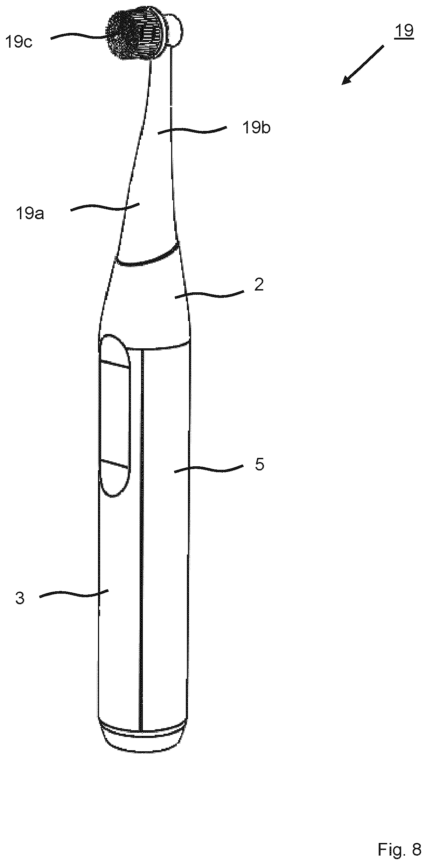

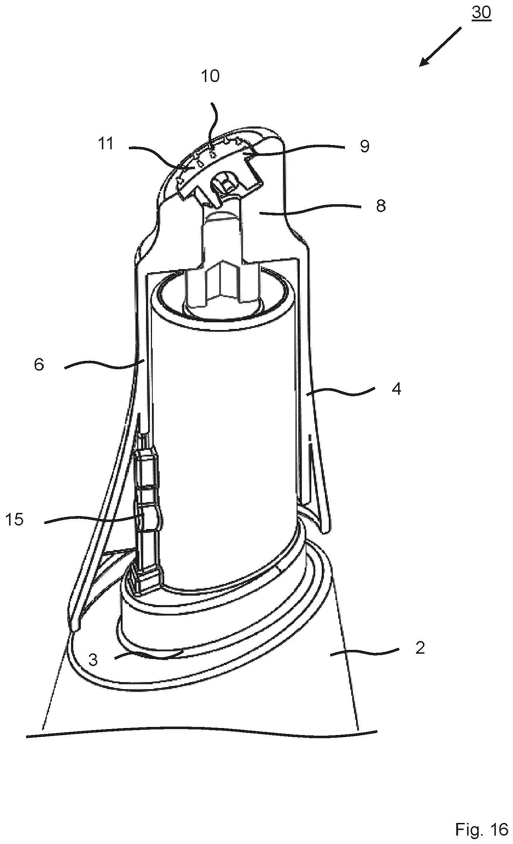

Im Folgenden werden unter Bezugnahme auf die Fig. 1 bis 7 sowie 9 bis 13 Beispiele für ein Microneedling-Handgerät erläutert, welche eine erfindungsgemäße schräg gestellte oder geneigte vordere Anwendungsfläche nicht aufweisen. Abgesehen von der in diesen Beispielen nicht erfindungsgemäß schräg gestellten Anwendungsfläche können die dort vorgesehen konstruktiven Ausgestaltungen einzeln oder in Kombination bei den erfindungsgemäßen Microneedling-Handgeräten mit schräger Anwendungsfläche vorgesehen sein, insbesondere bei den erfindungsgemäßen Microneedling-Handgeräten nach den Fig. 16 bis 18. Fig. 8 zeigt eine schematische perspektivische Darstellung einer bekannten elektrischen Zahnbürste.

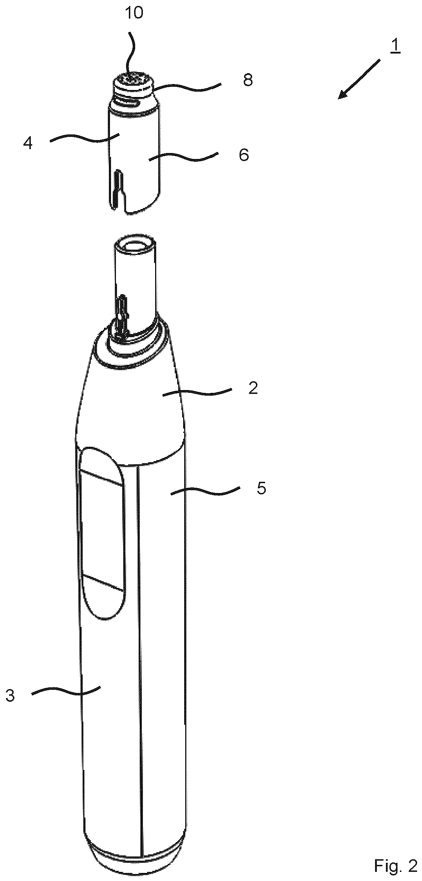

Fig. 1 bis 7 zeigen perspektivische Darstellungen für ein Microneedling-Handgerät 1, welches ein Gehäuse 2 aufweist, welches im gezeigten Beispiel mehrstückig ausgeführt ist. Das Gehäuse 2 ist mit einem hinteren Gehäuseteil 3 und einem vorderen Gehäuseteil 4 gebildet, die einem Antriebsmodul 5 und einer hieran lösbar angeordneten Hautapplikationseinrichtung 6 zugeordnet sind, mit dem wahlweise ein Stimulationsmodul 7 gebildet ist. Gemäß Fig. 2 ist die Hautapplikationseinrichtung 6 und somit wahlweise das Stimulationsmodul 7 von dem Antriebsmodul 5 abnehmbar und auf diese Weise wechselbar. Bei dem gezeigten Beispiel sind Antriebsmodul 5 und Hautapplikationseinrichtung 6 mit einer Steckverbindung lösbar verbunden.

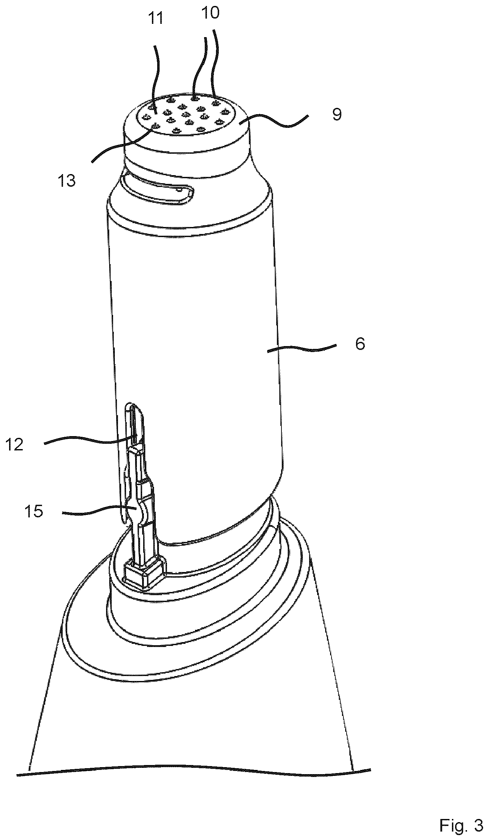

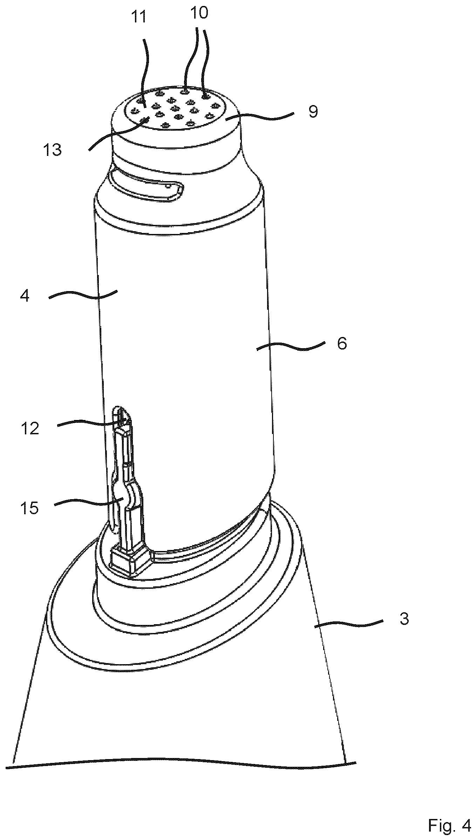

In dem hinteren Gehäuseteil 3, welches dem Antriebsmodul 5 zugeordnet ist, ist eine Antriebseinrichtung angeordnet, beispielsweise ein Elektromotor, mit der eine Antriebsbewegung oder -kraft bereitgestellt wird, die auf ein Microneedling-Applikator 8 der Hautapplikationseinrichtung 6 gekoppelt wird, um im Betrieb der Microneedling-Applikator 8 mittels der bereitgestellten Antriebskraft mit einer Wiederholfrequenz in Längsrichtung des Gehäuses 2 vor- und zurück zu bewegen. Auf diese Weise wird der Microneedling-Applikator 8, welcher mit einer Nadelplatte 9 gebildet ist, gemäß den Fig. 3 und 4 zwischen einer vorderen Arbeitsstellung (Fig. 3) und einer hinteren Arbeitsstellung (Fig. 4) repetierend verlagert. Auf diese Weise werden im Betrieb mehrere Applikationsnadeln 10, die an der Nadelplatte 9 im Bereich einer Anwendungsfläche 11 vorstehen, lokal auf einen zugeordneten Hautabschnitt angewendet (aufgedrückt) und wieder zurückgezogen. Hierzu sind die mehreren Applikationsnadeln 10 jeweils mit einer die Haut aufstechenden (scharfen) oder die Haut nicht aufstechenden (stumpfen) Nadelspitze ausgeführt.

Die Nadelplatte 9 mit den mehreren Applikationsnadeln 10 ist hierbei sowohl in der vorderen als auch in der hinteren Arbeitsstellung gemäß den Fig. 3 und 4 freiliegend, also insbesondere nicht von einem Abschnitt des Gehäuses 2 ganz oder teilweise umgeben und somit außerhalb eines Innenraums 12 des Gehäuses 2 angeordnet. Auf diese Weise kann der lokale Hautabschnitt, welcher mit den mehreren Applikationsnadeln 10 in Kontakt tritt, aufgrund des Vor- und Zurückbewegens des Microneedling-Applikators 8 mit der Nadelplatte 9 frei schwingen, was ein effizientes und möglichst schmerzfreies Anwenden auf der Haut unterstützt.

Im gezeigten Beispiel sind die mehreren Applikationsnadeln 10 in zugeordneten Öffnungen 13 im Bereich der Anwendungsfläche 11 an der Nadelplatte 9 angeordnet und hierin fixiert, beispielsweise mittels Klebens oder Lötens. In einer anderen Ausführungsform können die mehreren Applikationsnadeln 10 auf die Anwendungsfläche 11 aufgesetzt werden, beispielsweise mittels Aufschweißens. Alternativ können die Applikationsnadeln 10 mittels Spritzgussverfahren angeformt sein oder als Einlegebauteile integriert werden. Die mehreren Applikationsnadeln können oberhalb der Anwendungsfläche 11 alle eine im Wesentlichen gleiche Nadellänge oder unterschiedliche Nadellängen aufweisen, wobei hierbei Gruppen der mehreren Applikationsnadeln 10 von gleicher Nadellänge sein können.

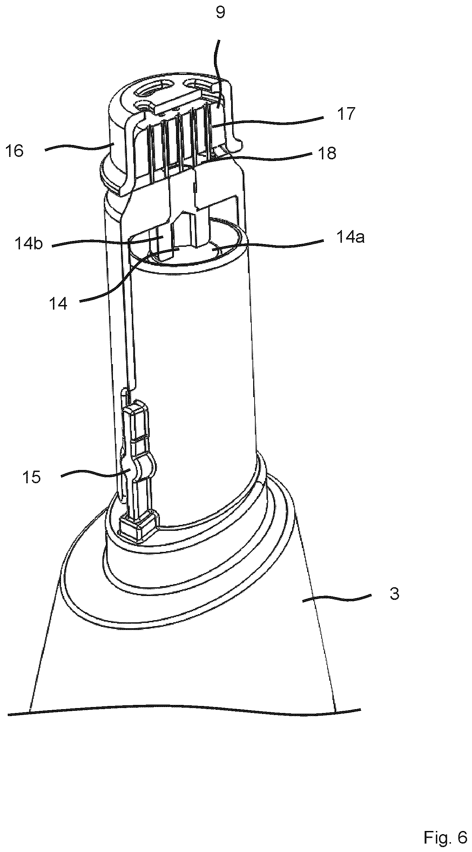

Bei dem gezeigten Beispiel in den Fig. 1 bis 7 ist die Hautapplikationseinrichtung 6, somit wahlweise das Stimulationsmodul 7, mittels Kopplung 14 aufgesteckt, die mit einem antriebsseitigen Kopplungsteil 14a und einem stimulationswerkzeugseitigen Kopplungsteil 14b gebildet ist. Die Kopplung 14 ist beispielweise als eine Magnetkopplung zwischen dem antriebsseitigen Kopplungsteil 14a und dem stimulationswerkzeugseitigen Kopplungsteil 14b ausgebildet (vgl. Fig. 6). Über die Kopplung 14 erfolgt die Einleitung der Antriebskraft / -bewegung zum Vor- und Zurückbewegen der Nadelplatte 9 mit den Applikationsnadeln 10.

Bei dem gezeigten Beispiel ist eine Verdrehsicherung 15 vorgesehen, welche das Stimulationswerkzeugmodul 6 gegen Verdrehen sichert.

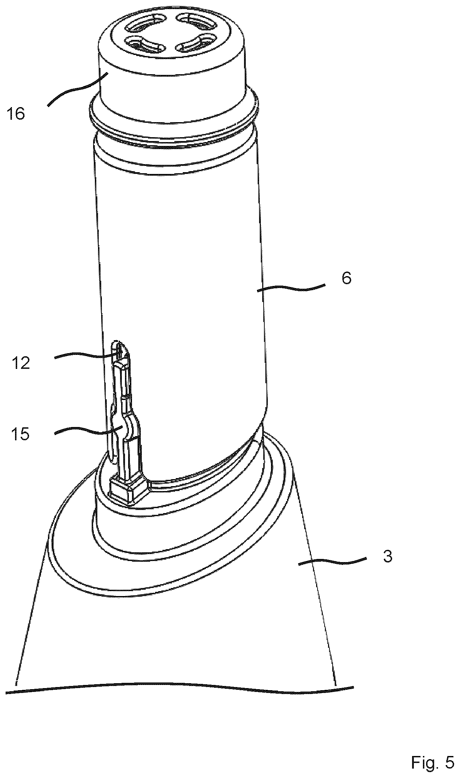

Gemäß Fig. 5 kann die Nadelplatte 9 mit den mehreren Applikationsnadeln 10 im Nichtbetrieb mittels einer Abdeck- oder Schutzkappe 16 abgedeckt werden, die lösbar aufgesetzt und so zum Betrieb abnehmbar ist. Beim gezeigten Beispiel ist die Abdeck- oder Schutzkappe 16 aufgesteckt.

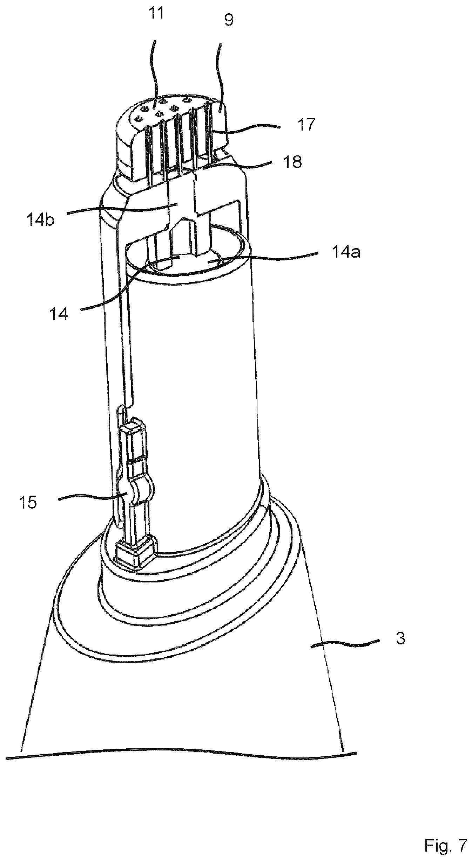

Fig. 6 und 7 zeigen den vorderen Abschnitt des Microneedling-Handgeräts 1 teilweise im Schnitt mit der und ohne die Abdeckkappe 16. Es ergibt sich, dass die mehreren Applikationsnadeln 10 in zugeordneten Bohrungen 17 der Nadelplatte 9 verlaufen und rückseitig zur Nadelplatte 9 an einem Nadelhalter 18 aufgenommen sind.

Fig. 8 zeigt eine schematische perspektivische Darstellung einer elektrischen Zahnbürste 19, bei der ein vorderer Gehäuseteil 19a mit einem Zahnbürstenaufsatz 19b gebildet ist, welcher anstelle der Hautapplikationseinrichtung 6 aufgesetzt ist und an die Antriebseinrichtung im hinteren Gehäuseteil 3 des Gehäuses 2 koppelt, so dass die Antriebskraft (Vor- und Zurückbewegung) genutzt wird, um eine Bürstenanordnung 19c im Betrieb zu bewegen. Hautapplikationseinrichtung 6 und Zahnbürstenaufsatz 19b können so wahlweise lösbar aufgesetzt und mit ein und demselben Antriebsmodul 5 betrieben werden, so dass entweder das Microneedling-Handgerät 1 oder die elektrische Zahnbürste 19 bereitgestellt sind.

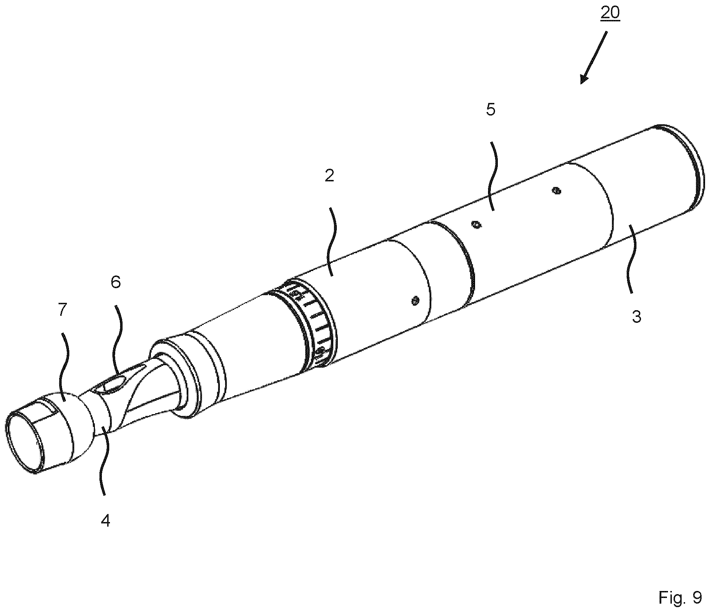

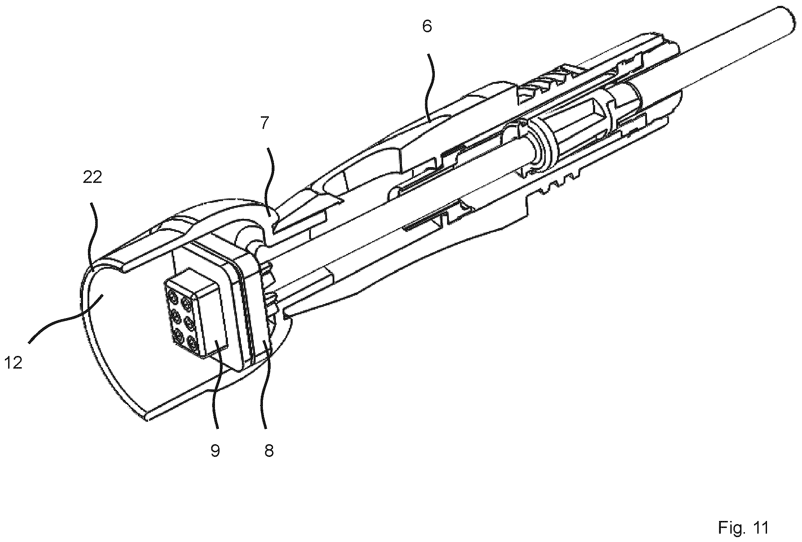

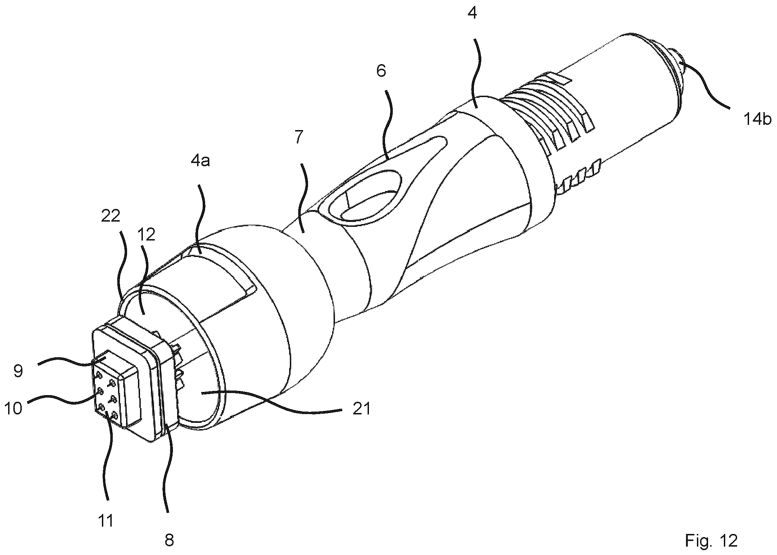

Fig. 9 bis 13 zeigen ein weiteres Microneedling-Handgerät 20, bei dem der Microneedling-Applikator 8 mit der Nadelplatte 9 durch eine vorderseitige Gehäuseöffnung 21 an der Hautapplikationseinrichtung 6 (Stimulationsmodul 7) zwischen einer eingefahrenen Nichtarbeitsstellung gemäß Fig. 9 bis 11 sowie ausgefahrenen Arbeitsstellungen gemäß den Fig. 11 und 12 verlagerbar ist.

Im Nichtbetrieb kann die Nadelplatte 9 der Hautapplikationseinrichtung 6 mit den hierauf angeordneten mehreren Applikationsnadeln 10 so hinter die Fläche der Gehäuseöffnung 21 zurückgefahren werden, derart, dass gemäß Fig. 9 sowohl Nadelplatte 9 wie auch Applikationsnadeln 10 vollständig hinter der Gehäuseöffnung 21 im Innenraum 12 des vorderen Gehäuseteils 4 angeordnet sind, welches bei der gezeigten Ausführungsform der Hautapplikationseinrichtung 6 zugeordnet ist. Am vorderen Gehäuseteils 4 ist die Gehäuseöffnung 21 an einem vorderen Abschnitt 4a gebildet.

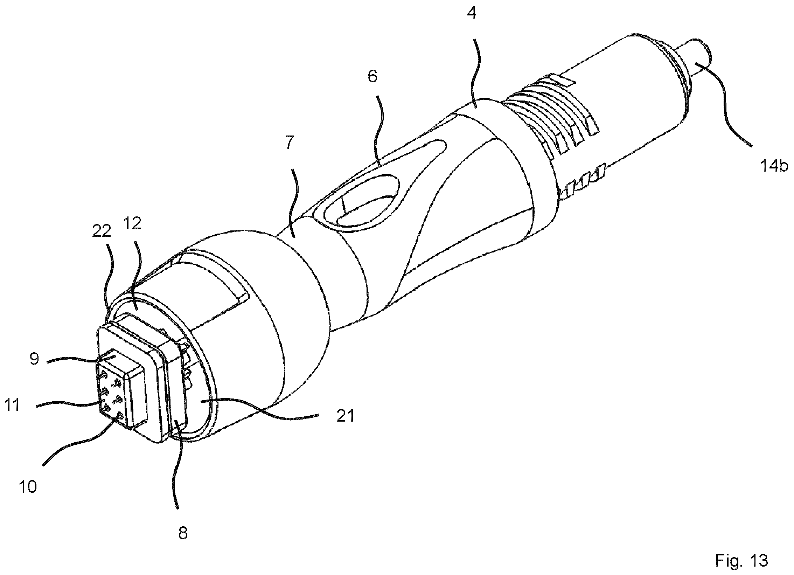

Im Betrieb fürs Microneedling wird die Nadelplatte 9 mit den mehreren Applikationsnadeln 10 gemäß den Fig. 12 und 13 zwischen der vorderen Arbeitsstellung (vgl. Fig. 12) und der hiergegenüber zurückgestellten hinteren Arbeitsstellung (vgl. Fig. 13) hin und her bewegt, also in Längsrichtung des Gehäuses 2 einem Arbeitshub entsprechend vor und zurück, so dass die Nadelplatte 9 mit den mehreren Applikationsnadeln 10 sowohl in der vorderen wie auch in der hinteren Arbeitsstellung in Bezug auf die Gehäuseöffnung 21 vor deren Fläche und somit außerhalb des Gehäuses 2 angeordnet ist, insbesondere außerhalb des vorderen Abschnitts 4a des vorderen Gehäuseteils 4, was wiederum ein Anregen des zu bearbeitenden Hautabschnitts zum freien Schwingen ermöglicht, also frei von einer Behinderung dieser Schwingung durch das Gehäuse 2, insbesondere einen die Gehäuseöffnung 21 umgebenden vorderen Gehäuserand 22. Bei dem gezeigten Beispiel ist im Betrieb im Wesentlichen der gesamte Microneedling-Applikator 8 vor der Gehäuseöffnung 21 angeordnet. Im Nichtbetrieb kann die Nadelplatte 9 dann in die eingefahrene Nichtarbeitsstellung gemäß Fig. 9 bis 10 verlagert werden.

Gemäß dem Beispiel in den Fig. 9 bis 13 ist die Nadelplatte 9 beim Hindurchführen durch die Gehäuseöffnung 21 umlaufend und durchgehend beabstandet vom Gehäuserand 22, also kontakt- und berührungsfrei.

Es kann vorgesehen sein, dass die Nadelplatte 9 innerhalb des vorderen Gehausteils 4 hinter der Gehäuseöffnung 21 mehrere unterschiedliche eingefahrene Nichtarbeitsstellungen einnehmen kann, die sich hinsichtlich ihres jeweiligen Abstands zur Gehäuseöffnung unterscheiden.

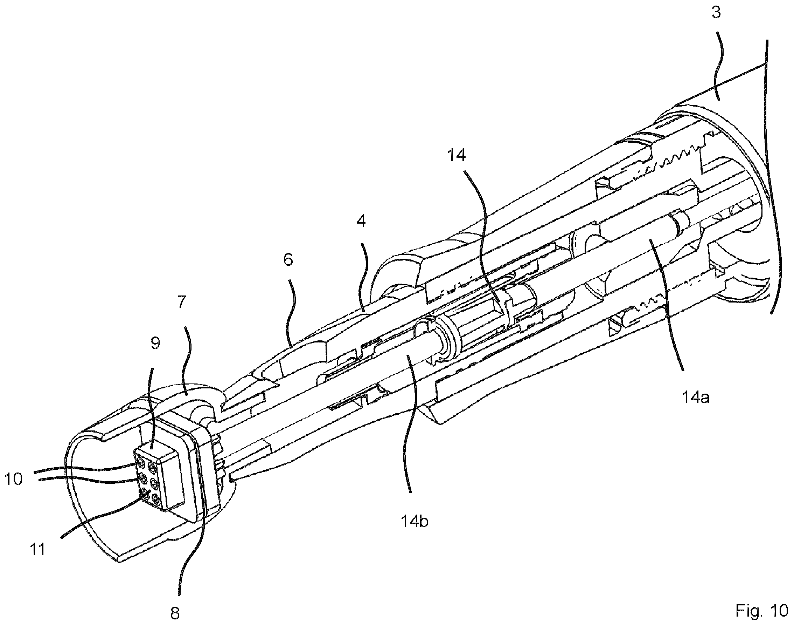

Im Bereich der Kopplung 14 ist das rückseitige, stimulationswerkzeugseitige Kopplungsteil 14b der Hautapplikationseinrichtung 6 gemäß Fig. 10 mit dem hier als Schaftbauteil ausgebildeten antriebsseitigen Kopplungsteil 14a (lösbar) verbunden, um die bereitgestellte Antriebskraft / -bewegung auf den Microneedling-Applikator 8 mit der Nadelplatte 9 einzukoppeln.

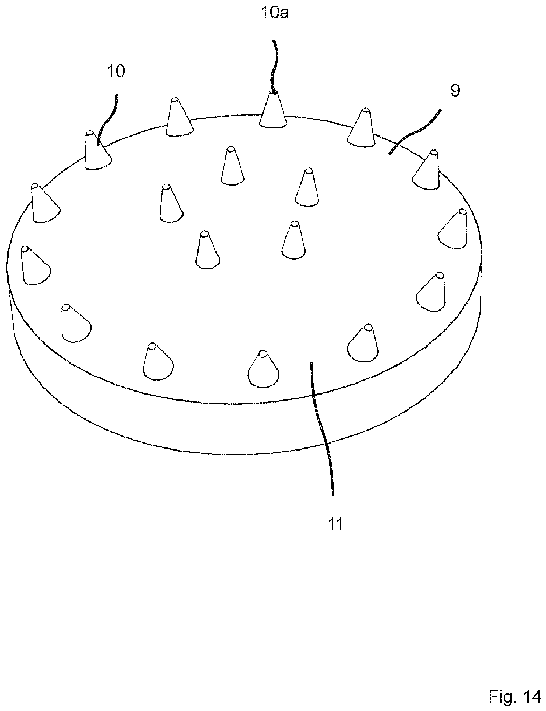

Fig. 14 zeigt eine schematische perspektivische Darstellung einer Ausführung der Nadelplatte 9 mit einer Anordnung der mehreren nicht-stechenden Applikationsnadeln 10 auf der Anwendungsfläche 11, die jeweils eine stumpfe Nadelspitze 10a aufweisen. Die mehreren Applikationsnadeln 10 sind bei dem gezeigten Ausführungsbeispiel mit einer Kegelform und einer abgeflachten Nadelspitze ausgebildet. Für gleiche Merkmale werden in den Fig. 14 bis 18 dieselben Bezugszeichen wie in den vorangehenden Figuren verwendet.

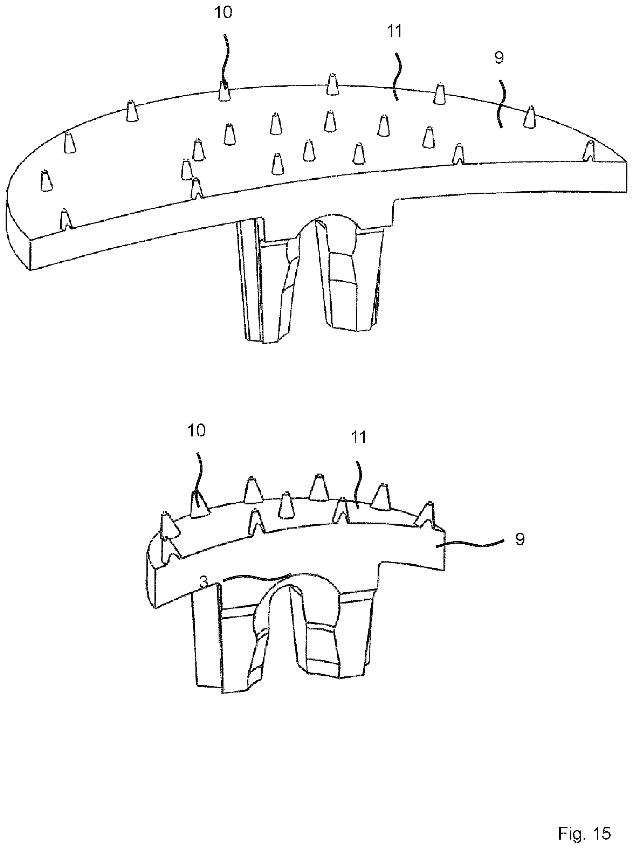

Fig. 15 zeigt eine schematische perspektivische Darstellung von Nadelplatten 9 unterschiedlicher Größe mit einer Anordnung von nicht-stechenden Applikationsnadeln 10 mit stumpfer Nadelspitze im Bereich der Anwendungsfläche 11 im Schnitt, wobei die Anwendungsfläche 11 konvex gekrümmt ist.

Die Fig. 16 bis 19 zeigen schematische perspektivische Darstellungen eines vorderen Abschnitts eines weiteren Microneedling-Handgerätes 30, bei dem die Anwendungsfläche 11 an der Nadelplatte 9 des Microneedling-Applikators 8 schräg gestellt oder geneigt ist in Bezug auf die Längsrichtung des Gehäuses 2, somit im gezeigten Beispiel auch schräg oder geneigt zur Bewegungsrichtung bei der Bewegung des Microneedling-Applikators 8 (mit der Nadelplatte 9) zwischen der vorderen und der hinteren Arbeitsstellung.

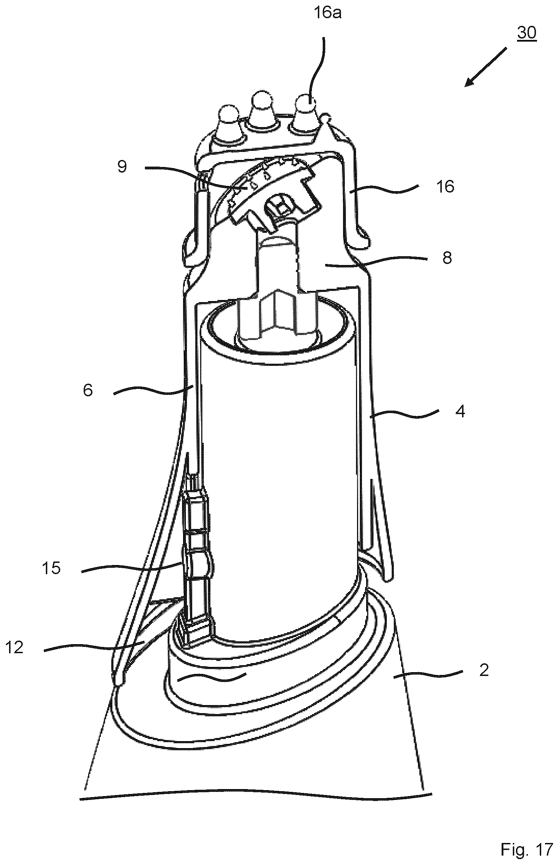

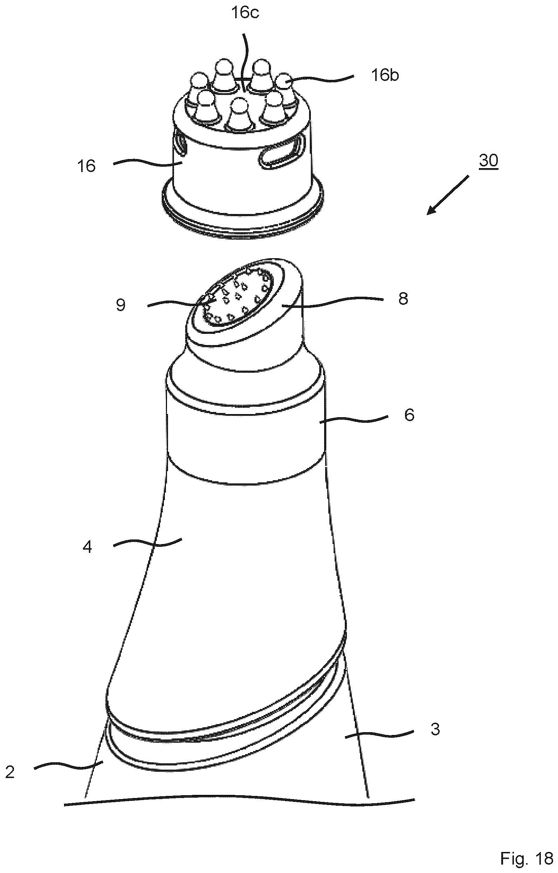

Die Fig. 17 und 18 zeigen das weitere Microneedling-Handgerät 30 mit lösbar aufgesetzter und abgenommener Abdeck- oder Schutzkappe 16. An der Abdeckkappe 16, welche im aufgesetzten Zustand (vgl. Fig. 17) die mehreren nicht-stechenden Applikationsnadeln 10 abdeckt, ist ein Funktionswerkzeug 16a angeordnet, welches bei dem dargestellten Ausführungsbeispiel mit einer Anordnung von Funktionselementen 16b gebildet ist, bei denen es sich beispielweise um Massageelemente handelt. Die Abdeckkappe 16 ist so als Multifunktionskappe ausgeführt. Beim dargestellten Ausführungsbeispiel sind die Funktionselemente 16b insbesondere im Bereich einer Frontfläche 16c der Abdeckkappe 16 ausgebildet.

Zusätzlich kann die Abdeckkappe 16, zumindest außen, eine geneigte Stirnfläche mit Funktionswerkzeug aufweisen. Auch wegklappbar ist denkbar, oder auch eine axial verschiebliche Hülse mit fehlender oder für das Funktionswerkzeug durchbrochener Deckfläche (vergleichbar einer Art Bohrkrone mit Werkzeugen am Umfang oder auf der Restdeckfläche), zu- / abschaltbar bspw. durch eine Drehkulisse-Kurvensteuerung per Dreh-Schiebe-Bewegung.

Andere Funktionswerkzeuge können vorgesehen sein, beispielsweise Silikonnoppen, Greif-Kneif-Werkzeuge aus elastischem Material und / oder abwechselnd gerichtete Stimulationswerkzeuge, die ihrerseits eine stech-ähnliche (Stechen simulierend) Wirkung auf der Haut erzielen und hierbei eine Hautspreizung bewirken. Alternative Funktionswerkzeuge zur Hautstimulierung können vorgesehen sein, die sich beispielweise bezüglich eines Spitzenradius oder -winkels von den vorgenannten unterscheiden.

Die Nadelplatte 9 kann in einer Ausgestaltung als Wendeplatte oder Wendeeinsatz ausgeführt sein, derart, dass unterschiedliche Applikatoren hieran jeweils wechsel- oder lösbar angeordnet werden können, um unterschiedliche (Microneedling-)Funktionen bereitzustellen oder einen Verschleiß des Applikators zu kompensieren. Es kann sich auch um Wechseleinsätze handeln, die je nach gewünschter Anwendung an der Nadelplatte 9 lösbar angeordnet werden. Wendeeinsätze können auf beiden Seiten Werkzeuge tragen. Die Wechseleinsätze können aus einem Sortiment oder einem abgestuften Satz von Applikatoreigenschaften auswählbar sein.

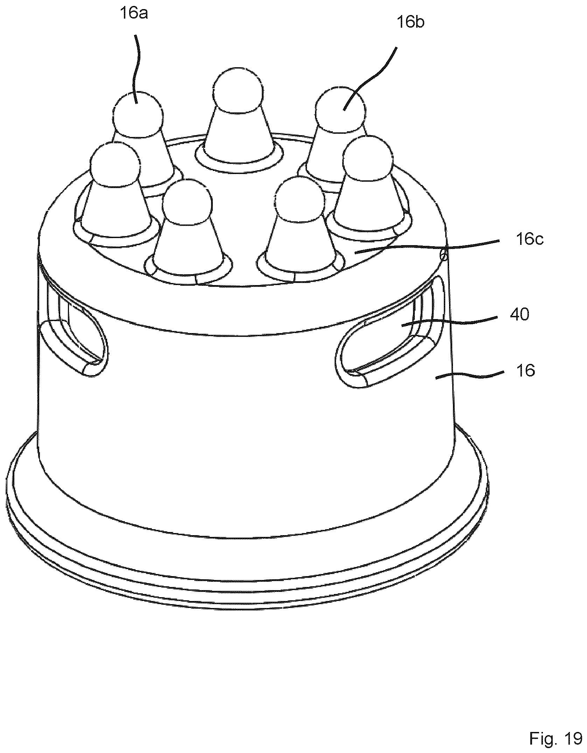

Fig. 19 zeigt eine vergrößerte perspektivische Darstellung der Schutz- oder Abdeckkappe 16 mit dem Funktionswerkzeug 16a. An der Abdeckkappe 16 sind Öffnungen 40 vorgesehen. Im gezeigten Beispiel sind die Öffnungen im Bereich einer Mantelfläche 41 der Abdeckkappe 16 angeordnet und bilden so seitliche Öffnungen. Alternativ oder ergänzend können solche Öffnungen bei anderen Ausführungsformen im Bereich der Frontfläche 16c angeordnet sein (vgl. Fig. 5). Zum Beispiel können die Öffnungen 40 dazu dienen, für die Nadelplatte 9 mit den mehreren Applikationsnadeln 10 bei aufgesetzter Abdeckkappe 16 eine Gassterilisation durchzuführen. Die in Fig. 18 und 19 gezeigten seitlichen Öffnungen 40 haben den Vorteil, dass der Eintritt von Staubpartikeln zu der Nadelplatte 9 und hierdurch verursachte Staubablagerungen gemindert sind.

Die in der vorstehenden Beschreibung, den Ansprüchen sowie der Zeichnung offenbarten Merkmale können sowohl einzeln als auch in beliebiger Kombination für die Verwirklichung der verschiedenen Ausführungen von Bedeutung sein.

## Patentansprüche

1. Microneedling-Handgerät (30), mit
- einem Gehäuse (2);
- einer Hautapplikationseinrichtung (6);
- einem Microneedling-Applikator (8), der an der Hautapplikationseinrichtung (6) gebildet und entlang einer Bewegungsrichtung vor und zurück bewegbar ist; und
- einer Nadelplatte (9), die an dem Microneedling-Applikator (8) gebildet ist und bei der über eine vorderseitige Anwendungsfläche (11) verteilt mehrere Applikationsnadeln (10) angeordnet sind;
wobei der Microneedling-Applikator (8) im Betrieb zwischen einer vorderen Arbeitsstellung und einer im Vergleich hierzu zurückgestellten hinteren Arbeitsstellung vor und zurück bewegbar ist; und
wobei die vorderseitige Anwendungsfläche (11) mit den hierauf verteilt angeordneten mehreren Applikationsnadeln (10), zumindest in der vorderen Arbeitsstellung, zu wenigstens einer Bezugsrichtung aus der folgenden Gruppe schräg gestellt ist: Längsrichtung des Gehäuses (2), Bewegungsrichtung des Microneedling-Applikators (8) beim Verlagern zwischen der vorderen und der zurückgestellten hinteren Arbeitsstellung und Bewegungsrichtung der Nadelplatte (9) beim Verlagern zwischen der vorderen und der zurückgestellten hinteren Arbeitsstellung.

2. Microneedling-Handgerät (30) nach Anspruch 1, **gekennzeichnet durch** eine Antriebseinrichtung, die in dem Gehäuse (2) angeordnet und eingerichtet ist, eine Antriebskraft bereitzustellen, wobei der Microneedling-Applikator (8) mit der Antriebseinrichtung verbunden ist, derart, dass der Microneedling-Applikator (8) mittels der Antriebskraft im Betrieb mit einer Wiederholfrequenz zwischen der vorderen Arbeitsstellung und der zurückgestellten hinteren Arbeitsstellung vor und zurück bewegbar ist.

3. Microneedling-Handgerät (30) nach Anspruch 1 oder 2, **dadurch gekennzeichnet, dass** beim Bewegen zwischen der vorderen Arbeitsstellung und der hinteren Arbeitsstellung, zumindest die mehreren Applikationsnadeln (10) der Nadelplatte (9), in der vorderen Arbeitsstellung und der hinteren Arbeitsstellung des Microneedling-Applikators (8) vollständig außerhalb des Gehäuses (2) und so freiliegend angeordnet sind.

4. Microneedling-Handgerät (30) nach mindestens einem der vorangehenden Ansprüche, **dadurch gekennzeichnet, dass** im Betrieb weiterhin zumindest die vorderseitige Anwendungsfläche (11) in der vorderen Arbeitsstellung und der zurückgestellten hinteren Arbeitsstellung des Microneedling-Applikators (8) vollständig außerhalb des Gehäuses (2) und so freiliegend angeordnet ist.

5. Microneedling-Handgerät (30) nach mindestens einem der vorangehenden Ansprüche, **dadurch gekennzeichnet, dass** bei dem Microneedling-Applikator (8) die Nadelplatte (9) frei von einem die Nadelplatte (9) zumindest teilweise umgreifenden Gehäuseabschnitt des Gehäuses (2) gebildet ist.

6. Microneedling-Handgerät (30) nach mindestens einem der vorangehenden Ansprüche, **gekennzeichnet durch** eine Schutzkappe, die am Gehäuse (2) lösbar und die Nadelplatte (9) abdeckend angeordnet ist, wobei an der Schutzkappe (16) außen ein Funktionswerkzeug angeordnet ist.

7. Microneedling-Handgerät (30) nach mindestens einem der vorangehenden Ansprüche, **dadurch gekennzeichnet, dass** ein vorderes Gehäuseteil (4), welches der Hautapplikationseinrichtung (6) zugeordnet ist, rückwärtig hülsenförmig gestaltet ist und einen hinteren Gehäuseteil (3) zumindest teilweise umschließt, welcher der Antriebseinrichtung zugeordnet ist.

8. Microneedling-Handgerät (30) nach mindestens einem der vorangehenden Ansprüche, **dadurch gekennzeichnet, dass** die Hautapplikationseinrichtung (6) an dem Gehäuse (2) lösbar angeordnet ist.

9. Microneedling-Handgerät (30) nach Anspruch 8, **dadurch gekennzeichnet, dass** die Antriebseinrichtung mit einer Antriebseinrichtung für eine elektrische Zahnbürste gebildet ist, welche eingerichtet ist, hieran wahlweise die Hautapplikationseinrichtung (6) oder einen mittels der Antriebseinrichtung betreibbaren Zahnbürstenaufsatz lösbar zu koppeln.

10. Microneedling-Handgerät (30) nach mindestens einem der vorangehenden Ansprüche, **dadurch gekennzeichnet, dass** der Microneedling-Applikator (8) durch eine Öffnung (21) an einem vorderseitigen Gehäuseende des Gehäuses (2) hindurch in eine im Vergleich zur zurückgestellten hinteren Arbeitsstellung weiter zurückgestellte Nichtarbeitsstellung verlagerbar ist, in welcher zumindest die vorderseitige Anwendungsfläche der Nadelplatte (9) gegenüber der Öffnung (21) zurücksteht.

11. Microneedling-Handgerät (30) nach Anspruch 10, **dadurch gekennzeichnet, dass** die Nadelplatte (9) beim Verlagern durch die Öffnung (21) hindurch ein- oder mehrseitig von einem die Öffnung (21) umgebenden Rand (22) beabstandet ist.

12. Microneedling-Handgerät (30) nach Anspruch 10 oder 11, **dadurch gekennzeichnet, dass** die Nadelplatte (9) in der weiter zurückgestellten Nichtarbeitsstellung beabstandet von die Nadelplatte (9) umgebenden Gehäuseabschnitten des Gehäuses (2) freiliegend angeordnet ist.

13. Microneedling-Handgerät (30) nach mindestens einem der vorangehenden Ansprüche, soweit auf Anspruch 2 rückbezogen, **gekennzeichnet**durch ein wiedervendbares Antriebsmodul (4), in welchem die Antriebseinrichtung angeordnet ist, und ein Einwegmodul (5), welches mit dem Antriebsmodul lösbar verbunden ist und in dem die Hautapplikationseinrichtung (6) angeordnet ist.

14. Microneedling-Handgerät (30) nach mindestens einem der vorangehenden Ansprüche, soweit auf Anspruch 2 rückbezogen, **dadurch gekennzeichnet, dass** die Antriebseinrichtung eingerichtet ist, der Microneedling-Applikator (8) mittels der hierauf eingeleiteten Antriebskraft im Betrieb mit einer Wiederholfrequenz zwischen etwa 10 Hz und etwa 200 Hz vor und zurück zu bewegen.

15. Hautapplikationseinrichtung (6) mit einem Gehäuse, die zum Ausbilden eines Microneedling-Handgeräts (30) nach mindestens einem der vorangehenden Ansprüche an eine Antriebseinrichtung koppelbar ist, mit:
- einem Microneedling-Applikator (8), der an der Hautapplikationseinrichtung (6) gebildet ist, derart, dass der Microneedling-Applikator (8) im Betrieb entlang einer Bewegungsrichtung vor und zurück bewegbar ist; und
- einer Nadelplatte (9), die an dem Microneedling-Applikator (8) gebildet ist und bei der über eine vorderseitige Anwendungsfläche (11) verteilt mehrere Applikationsnadeln (10) angeordnet sind;
wobei der Microneedling-Applikator (8) zwischen einer vorderen Arbeitsstellung und einer im Vergleich hierzu zurückgestellten hinteren Arbeitsstellung vor und zurück bewegbar ist und
wobei die vorderseitige Anwendungsfläche (11) mit den hierauf verteilt angeordneten mehreren Applikationsnadeln (10), zumindest in der vorderen Arbeitsstellung, zu wenigstens einer Bezugsrichtung aus der folgenden Gruppe schräg gestellt ist: Längsrichtung des Gehäuses, Bewegungsrichtung des Microneedling-Applikators (8) beim Verlagern zwischen der vorderen und der zurückgestellten hinteren Arbeitsstellung und Bewegungsrichtung der Nadelplatte (9) beim Verlagern zwischen der vorderen und der zurückgestellten hinteren Arbeitsstellung.

16. Artikel, mit
- einer Antriebseinrichtung, die in einem Gehäuse angeordnet ist, welches wahlweise ein Handstück ausbildend ausgeführt ist;
- einem Zahnbürstenaufsatz, welcher zum Ausbilden einer elektrischen Zahnbürste mit dem Gehäuse lösbar und so an die Antriebseinrichtung koppelnd verbunden werden kann; und
- einer Hautapplikationseinrichtung (6), welche zum Ausbilden eines Microneedling-Handgeräts (30) mit dem Gehäuse lösbar und so an die Antriebseinrichtung koppelnd verbunden werden kann.
